(19) Europäisches Patentamt
European Patent Office
Office européen des brevets

(11) EP 4 466 034 B1

(12) **EUROPEAN PATENT SPECIFICATION**

(45) Date of publication and mention of the grant of the patent:
**20.05.2026 Bulletin 2026/21**

(21) Application number: **23703866.6**

(22) Date of filing: **18.01.2023**

(51) International Patent Classification (IPC):
***A61L 26/00*** *(2006.01)* ***A61K 38/57*** *(2006.01)*
***A61K 9/00*** *(2006.01)* ***A61K 47/00*** *(2006.01)*

(52) Cooperative Patent Classification (CPC):
**A61L 26/0019; A61K 9/06; A61K 47/38; A61L 26/0047; A61L 26/0076; A61L 26/008**

(86) International application number:
**PCT/IB2023/050421**

(87) International publication number:
**WO 2023/139492 (27.07.2023 Gazette 2023/30)**

(54) **NEW SYSTEM FOR THE TOPICAL PHARMACOLOGICAL RELEASE OF AN ACTIVE INGREDIENT**

NEUES SYSTEM ZUR TOPISCHEN PHARMAKOLOGISCHEN FREISETZUNG EINES WIRKSTOFFS

NOUVEAU SYSTÈME DE LIBÉRATION PHARMACOLOGIQUE TOPIQUE DE PRINCIPE ACTIF

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **20.01.2022 IT 202200000884**
**14.02.2022 IT 202200002618**

(43) Date of publication of application:
**27.11.2024 Bulletin 2024/48**

(73) Proprietor: **Università Degli Studi Di Padova**
**35122 Padova (PD) (IT)**

(72) Inventors:
- **PONTISSO, Patrizia**
  **35123 Padova PD (IT)**
- **MORPURGO, Margherita**
  **35137 Padova PD (IT)**
- **FADINI, Gian Paolo**
  **35020 Albignasego PD (IT)**
- **ALBIERO, Mattia**
  **35125 Padova PD (IT)**
- **BIASIOLO, Alessandra**
  **35030 Rubano PD (IT)**
- **QUARTA, Santina**
  **35030 Villaguattera - Rubano PD (IT)**
- **RUVOLETTO, Mariagrazia**
  **30030 Vigonovo VE (IT)**
- **TURATO, Cristian**
  **35010 Villafranca Padovana PD (IT)**
- **VILLANO, Gianmarco**
  **35127 Padova PD (IT)**

(74) Representative: **Di Giovine, Paolo et al**
**Società Italiana Brevetti S.p.A.**
**Piazza di Pietra, 38-39**
**00186 Roma (IT)**

(56) References cited:
**WO-A1-2007/115860 US-A1- 2016 317 422**

- **KIM HYE SUNG ET AL: "Advanced drug delivery systems and artificial skin grafts for skin wound healing", ADVANCED DRUG DELIVERY REVIEWS, ELSEVIER, AMSTERDAM , NL, vol. 146, 31 December 2018 (2018-12-31), pages 209 - 239, XP085893408, ISSN: 0169-409X, [retrieved on 20181231], DOI: 10.1016/J.ADDR.2018.12.014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- FUMAKIA MIRAL ET AL: "Nanoparticles Encapsulated with LL37 and Serpin A1 Promotes Wound Healing and Synergistically Enhances Antibacterial Activity", MOLECULAR PHARMACEUTICS, vol. 13, no. 7, 5 July 2016 (2016-07-05), US, pages 2318 - 2331, XP055956827, ISSN: 1543-8384, DOI: 10.1021/acs.molpharmaceut.6b00099
- TEOLI ET AL: "Wet sol-gel derived silica for controlled release of proteins", JOURNAL OF CONTROLLED RELEASE, ELSEVIER, AMSTERDAM, NL, vol. 116, no. 3, 1 December 2006 (2006-12-01), pages 295 - 303, XP005787054, ISSN: 0168-3659, DOI: 10.1016/J.JCONREL.2006.09.010
- FADINI GIAN PAOLO ET AL: "The molecular signature of impaired diabetic wound healing identifies serpinB3 as a healing biomarker", vol. 57, no. 9, 1 September 2014 (2014-09-01), Berlin/Heidelberg, pages 1947 - 1956, XP055956241, ISSN: 0012-186X, Retrieved from the Internet <URL:https://link.springer.com/content/pdf/10.1007/s00125-014-3300-2.pdf> DOI: 10.1007/s00125-014-3300-2

## Description

### Technical field

The present invention relates to the field of the formulation of bioactive compounds. More specifically it relates to the use of wet silica-based polymers obtained with the sol-gel technology for the incorporation and prolonged release of an active ingredient, preferably of a protein, for topical use, for example in the treatment of chronic ulcers and for the improvement of the trophism of skin appendages. Under chronic ulcer a skin continuous lesion is meant which does not heal and does not progress through the usual phases and usual healing time periods and the most common chronic ulcers include the lower limb ulcers, the diabetic ulcers and the pressure ulcers. The composition of the invention has several advantages, in particular it has a protein slow release and a long permanence in the application point, by avoiding that the composition drips or dries up in contact with air.

### State or art

The chronic ulcers, differently from the acute ulcers which can heal in few days or weeks, can last for months or years. This condition burdens physically and psychologically on the individual and represents a management problem for the health care system. The ulcers are often consequence of extrinsic factors, such as for example a trauma, and appear together with intrinsic factors, such as for example a sensitivity reduction or a load increase. Frequent pathological conditions which can lead to the onset of ulcers are the chronic venous insufficiency, the diabetic disease, the infections and the peripheral vascular diseases. In particular, the foot chronic ulcer is one of the most invalidating complications of diabetes, which often leads to amputation, with consequent disability, and it is burdened with significant mortality. Other less frequent cases are due to genetic diseases (prolidase deficiency) autoimmune diseases (systemic lupus erythematosus, scleroderma, dermatomyositis, periarteritis nodosa), chronic intoxications (alcoholism, drug addictions), myelolymphoproliferative diseases (leukemiae, polyglobulia, lymphomas, haemolytic anaemias, thrombocythemia rubra, multiple myeloma).

The healing of wounds is a complex process involving several tissues and types of cells, characterized by several and coordinated phases thereamong coagulation, fibrogenesis, formation and regression of granulation tissue, angiogenesis and inflammation. The alterations of each one of above events can delay healing and/or favour the wound complications.

The mechanisms responsible for the delayed healing of ulcers are not yet wholly clear. However, in a recent proteomic study performed on samples of ulcerative tissue of diabetic patients, SerpinB3 (SB3) protein was identified as a biomarker of a quicker wound healing. SerpinB3 is a protease inhibitor, involved in the activation of fibrogenesis and angiogenesis, two key processes in the wound resolution, and it resulted to be more expressed in the ulcers of patients with ulcer quicker resolution than the slow resolution ones (Fadini 2014, doi:10.1007 /s00125-014-3300-2).

However, the topical administration of SB3 in an open ulcer has some technical difficulties due to the protein nature of this agent. In fact, the ulcer environment usually is aggressive against proteins - which are subject to secondary inactivation to proteolytic digestion and/or to physical denaturation - and it can reduce quickly the local levels of SB3, unless a constant intake of functionally active protein is provided. In order to obtain this, a prolonged release of the bioactive agent in the action site is preferable. Moreover, the viscoelastic properties of the formulation should allow a prolonged permanence in the application point, without dripping or drying up. At last, the vehicle should be capable of preserving the stability over time of the active agent to prolong the preservation duration.

Although the topical administration of proteins can be useful in some pathologies, up to now little effort has been dedicated to the development of formulations suitable to this route (Aufenvenne K, et al. Am J Hum Genet. 2013;93(4):620-30). Most strategies described in literature for vehiculating proteins, in fact, are aimed at the parental application or the local administration for the purpose of a systemic effect. Besides, many optimized solutions for the parental uses (liposomes, micelles, or inclusion in hydrogels) could be suitable to the external application, on condition that the features for releasing the active element and the viscoelastic properties of the administered formulation are suitable for this use.

The use of formulations in hydrogel (crosslinking hydrophilic polymeric structures which can absorb huge amounts of water solutions) can be particularly suitable for this purpose. In fact, the hydrogels, which can be obtained by using a wide variety of properties of the polymeric materials, can both stabilize the proteins and generate systems with adequate viscoelastic properties (Faisal Raza, Pharmaceutics 2018;10(1):16; Allan S.Hoffman. Advanced Drug Delivery Reviews 2012, 64 (Suppl): 18-23; Susana Simões, et al. Journal of Biomaterials and Nanobiotechnology, 2012, 3, 185-199). However, not all of them are capable of generating a slow and prolonged release of proteins over time, required to confer their constant intake on the application site.

A possible solution to obtain a prolonged release of active proteins from a semi-solid hydrophilic formulation consists in incorporating them in wet silica sol-gel-based vehicles, as described in the patent WO2007115860A1. The

silica sol-gel is a type of highly porous silica polymer obtained synthetically from precursors of silicon alkoxide. When in aqueous environment, the silica polymers degrade slowly in silicic acid - a component of the mineral waters - which is biocompatible and not toxic.

The polymerization of silica sol-gel is a delicate process which takes place in aqueous/alcoholic environment, compatible with the stability of many proteins. Should the polymerization be performed in presence of bioactive agents, these remain physically incorporated inside the just generated polymeric network. Many proteins, when incorporated in the silica sol-gel, keep their tertiary and quaternary structure and are released slowly in aqueous media. The release takes place after polymer diffusion and erosion phenomena, the speed thereof depends upon several factors, thereamong the matrix hydration level. In particular, until they are released by the gel, the incorporated proteins are protected against degradation physiological processes.

Besides, the wet silica sol-gel polymers are not spreadable. In fact, in direct contact with air, they dry up quickly and undergo an irreversible syneresis, which reduces the sizes of the polymer pores and blocks the protein release.

Then, it is necessary to combine these factors in a composition which allows a lasting permanence on the application point, without dripping or drying up.

## Summary of the invention

The technical problem placed and solved by the present invention is to provide a silica sol gel-based composition for topical use for the controlled release of an active ingredient, which allows a lasting permanence in the application point, without dripping or drying up in contact with air.

The solution proposed by the authors of the present invention is represented by a composition for topical use comprising or consisting of a wet silica sol gel, characterized in that it has an active ingredient, incorporated in said wet silica sol gel and said wet silica sol gel is diluted in a cellulose polymer hydrogel, in particular a hydroxypropylmethylcellulose hydrogel, preferably containing glycerol. The presence of such hydroxypropylmethylcellulose hydrogel is particularly advantageous since it allows the composition to be applied by avoiding drippings and without drying up in contact with air, moreover, the not loaded nature of this polymer avoids possible charge interaction with the vehiculated protein which could influence the stability and release of the same.

The authors of the present invention have surprisingly found that the herein described composition is particularly useful in the treatment of skin ulcers and could be used at clinical level, especially for the foot chronic ulcer or other part of the lower limbs, one of the most invalidating complications of diabetes, which often leads to amputation and it is burdened by a significant morbidity and mortality.

The present invention firstly relates to a composition as claimed comprising or consisting of a wet silica sol gel, wherein an active ingredient is incorporated in said wet silica sol gel, preferably said active ingredient is a protein.

The present invention further relates to a method for the preparation of a composition comprising or consisting of a wet silica sol gel, wherein an active ingredient is incorporated in said wet silica sol gel, preferably said active ingredient is a protein.

As defined in the claims, the method, the invention relates to, comprises the following steps:

i) preparation of an activated sol;
ii) mixing said activated sol with a solution of an active ingredient so as to obtain a wet silica sol-gel incorporating said active ingredient;
iii) washing the wet gel to replace the dispersing solution with a suitable buffer, after, or not, crushing it to generate a suspension of coarse wet gel particles;
iv) dilution of said sol-gel obtained at point iii) in a cellulose polymer hydrogel.

Additional advantages and/or embodiments of the present invention will be evident from the following detailed description.

## Brief description of figures

The present invention and the following detailed description of the preferred embodiments can be better understood with reference to the following figures:

**Figure 1. Effect of incorporation in the hydrogel of wet silica gel sol gel on the stability of SB3.** Circular dichroism spectra of human SB3 (h-SB3) recorded A) at 25°C in solution (solid line) or in gel (dotted line); B) curves of thermal denaturation of protein in buffer solution -100 mM phosphate pH 6.0 (dotted line), or incorporated in the wet silica gel sol-gel (solid line).

**Figure 2. Western blot analysis of SerpinB3 subjected to different treatments.** From left to right: native SB3; SB3

after incubation in solution for 1 h at 37°C with the pronase proteolytic enzyme; SB3 released by wet silica sol-gel (8% SiO2 p/p) previously incubated for 1 hour at 37°C with a pronase solution; SB3 released by wet silica sol-gel (8% SiO2 w/w) previously treated for 1 h at 37°C with buffer without pronase.

**Figure 3. Release of SB3 from wet silica sol-gel (8% SiO2 w/w).** The gels were previously crushed manually to generate a suspension of coarse particles. (A) Percentage of SB3 released (Mt/Mtot%) as a function of time (h) of immersion in buffer at 37%C. (B) Percentage of SB3 released (Mt/Mtot%) as a function of the square root of time. The linear correlation demonstrates that the protein release takes place through a diffusion process. The gels were loaded with protein at SB3 5 and 50 μg/mL. C) Scatter assay on MDCK cells. The solution of release from gel at SB3 50 μg/mL induced the time-depending scattering of strictly cohesive colonies of MDK cells, similar to the effect of recombinant SB3, used as positive control (CTR+). Negative control= CTR-

**Figure 4. SB3 formulated in wet silica sol gel and hydrogel of HPMC/glycerol in the treatment of the diabetic ulcers.** A) Experimental design for inducing diabetes, execution and treatment of wound. B) healing profiles of wounds in animals treated with vehicles only (B1) and with SB3 formulated in the different vehicles (B2). C) Representative images of diabetic ulcers at basal time (0) and 3 days after the wound. D) areas below the curve calculated by the panel B2). A decrease in AUC means quicker healing; *$p<0.05$ after 2-route Anova. $SiO_2$= wet silica sol-gel SiO2 8% w/w.

**Figure 5. Hair trophism after inducing skin ulcers.** Example of hair regeneration on day 1 and on day 10 as from inducing 4 skin ulcers on the mouse back. On the right the scheme of the performed treatments is represented, consisting in: 1= no treatment; 2= SerpinB3 in wet silica sol-gel; 3= SerpinB3 + Metolose; 4= vehicle only (wet silica sol-gel).

**Figure 6. Release profiles of BSA-FITC** (385 μg/mL) from wet silica sol-gel (SiO2 8% p/p) suspended in 3% HPMC hydrogel (◦) or in buffer (•)

**Figure 7. Water evaporation from 3% wN HPMC hydrogel depending upon the amount of present glycerol.** Percentage of residual H2O in the HPMC fraction mixed with several amounts of glycerol, after 5 h and 24 h of exposure to air.

**Figure 8. Invasion and migration assay.** The human fibroblasts were sowed at 2,000 cells/well and incubated with recombinant SB3 at a concentration of 200 ng/ml. Invasion (Inv) and migration (Mig) were measured after 15 hours from the cell seeding. **p=0.0043, *p=0.0003.

**Figure 9. Murine SerpinB3 (mSB3) induces proliferation and invasion on murine fibroblasts.** The mouse fibroblasts were treated with several concentrations of mSB3. A) Proliferation rate vs not treated cells dopo 72 hours of treatment with mSB3. B) Invasion rate vs not treated cells after 15 hours of treatment.

## Detailed description

Several embodiments of the invention will be described hereinafter. It is to be meant that the features of the various embodiments can be combined, if compatible. Generally, the subsequent embodiments will be described only with respect to the differences with those described previously.

As anticipated above, the present invention firstly relates to a composition suitable for topical use comprising or consisting of a wet silica sol gel containing an active ingredient, preferably a protein such for example protein SerpinB3, or a fragment or a variant thereof, incorporated therein. Said composition is according to the claims.

Under the term "sol" in the present description one relates to a colloidal suspension of solid particles in a liquid, in particular wherein the particles have dimensions ranging from 1 nm and 1 μm.

In any point of the present description under the term "active ingredient" or even "active principle" a substance provided with a specific pharmacological activity is meant including all substances explicating in the formulation a specific activity, for example with therapeutic or preventive effect.

Under the term silica "sol-gel" in the present description a product obtained by means of a process called "sol-gel process" is meant. In the sol-gel synthesis, a silica "sol", consisting of a hydroalcoholic mixture containing a mixture of partially hydrolyzed alkoxysilanes, in presence, or not, of silica polymers with low molecular weight generated by condensation of the first ones and having linear or branched conformation, is made to evolve through reactions of hydrolysis and condensation, until the formation of a continuous inorganic lattice containing an interconnected liquid phase (gel). After suitable thermal treatments the solvent contained in the gel can be made to evaporate and the material is so converted in a solid product. According to the used technique, the products obtained through the sol-gel process can be deposited as surface coating, or obtained as massive products.

As far as the silica sol gel is concerned, the starting point is constituted by an alkoxide or by a silanol which causes hydrolysis and condensation reactions often catalysed by acid or basic environments. In the hydrolysis reaction there is the replacement of an alkoxide group -OR with a hydroxyl group -OH. In the subsequent condensation reactions, there is the formation of siloxane bonds (Si-O-Si) with formation of water or alcohol

=Si-OR + H2O $\rightarrow$ =Si-OH + ROR (hydrolysis)

=Si-OH + =Si-OR $\rightarrow$ =Si-O-Si= + ROH (condensation)

=Si-OH + =Si-OH $\rightarrow$ =Si-O-Si= + H2O (condensation).

At the end of the hydrolysis and condensation reactions, a three-dimensional polymeric structure is obtained, soaked in a liquid consisting of water and outgoing alcohols and by the possible solvent introduced in the process. The removal of the liquid outside the polymeric lattice can be implemented by drying or under super-critical conditions, which can be followed by an additional thermal treatment, to favour, if needed, additional polycondensations and to improve the mechanical properties and the structural stability of the material through sintering. In case the solvent removal is not performed, or this is exchanged, through a washing step, with a different liquid, but liquid mixable with the first one, the sol-gel is defined as wet silica sol-gel. The possibility of varying the solvent type, the proportions between metal alkoxide and water in the hydrolysis reaction, the ionic force of the reaction medium, the catalyst type, leads to the formation of products having different properties which undoubtedly represents one of the major advantages offered by the sol-gel process.

Under the word "incorporated" in the present description it is meant that the molecule is inside the silica gel, in particular it is physically incorporated/entrapped in said silica gel.

In the present invention, said silica gel is a silica sol gel,

Said silica sol gel is a wet silica sol gel.

According to an additional embodiment, compatibly with all herein described embodiments, the composition, the invention relates to, is characterized in that it has an amount of water in said sol-gel ranging from 76% to 98% w/v (weight/volume), preferably from 80% to 94% w/v.

In an embodiment, the water content in said sol-gel is in a range from 80% to 94% w/v.

In an embodiment, said active ingredient is selected among a biological macromolecule, preferably a protein, or a peptide.

Examples of biological macromolecules are lipids, proteins thereamong for example enzymes, antibodies, monoclonal antibodies, nucleic acids thereamong DNA and RNA, carbohydrates, for example polysaccharides.

In a preferred embodiment, said active ingredient is a protein, in a still more preferred embodiment said active ingredient is SerpinB3 protein, or a fragment or a variant thereof.

In a preferred embodiment, said protein is Serpin B3 protein or a fragment thereof or a variant thereof with the same function, preferably said protein is physically incorporated in said silica gel.

SerpinB3 is a member of serin-protease (Serpine) inhibitor family, expressed physiologically in the squamous epithelia (Kato et al, Anticancer Res. 1996; 199616(4B):2149-2153). Known as Squamous Cell Carcinoma Antigen, SCCA1, belonging to the ov-serpins, it is usually expressed in the squamous epithelia, but it is hyper-expressed in the neoplastic cells of epithelial origin and hepatocarcinoma. The serpins are a family of serin protease inhibitors and they are involved in many biological functions and in the processes for controlling the cellular homeostasis. SerpinB3 can influence mobility, invasiveness proliferation and cellular death itself.

The word "fragment" in the present description relates to short segments of the peptide structure, typically from 5 to 15 residues, and it does not include the side chains.

The word "variant" in the present description relates to a member of a set of highly similar proteins which originate from one single gene or family of genes and they are the result of genetic differences. In the compositions of the present invention a fragment or variant of the wild type form of SerpinB3 could be used which have the same function, that is which are inhibitor of serin-protease, for example shorter fragments could be used comprising for example the functional domain only, or variants with an identity sequence comprised for example between 90 and 99%, wherein the differences with respect to the wild type form increase or do not alter the protein function. In a preferred embodiment the wild type human SerpinB3, or isoforms thereof, will be used, in particular having the aminoacidic sequence corresponding to the sequences of the data bank UniProtKB - P29508 (SPB3_HUMAN) as available at time of filing.

In another embodiment, the ratio between silica ($SiO_2$) and the solution in which it is immersed is in a range from 2% to 20% w/v, preferably from 4% to 15% w/v.

In an embodiment, the concentration of silica ($SiO_2$) contained in the composition is in a range from 4% to 15% w/v.

In a preferred embodiment, said incorporated active ingredient is present in a concentration in a range from 0.005 to 10.0 mg/ml of gel.

The composition, the invention relates to, further comprises a hydrated semisolid vehicle.

Under the term "hydrated semisolid vehicle" in the present description one relates to three-dimensional systems consisting of a polymer (dispersed phase) englobing the dispersing phase, in this case water, in huge amounts, also called "hydrogels". The hydrogels are cross-linked polymeric structures capable of absorbing a high amount of water or biological fluids. They can form different hydrogels of natural compounds, such as the case of agar agar and various polysaccharide

molecules, but even synthetic compounds such as polyacrylamide. The presence of several hydrophilic groups in the dispersed molecule is fundamental. Considering the nature and composition of hydrogels, these are commonly defined hydrocolloidal.

The hydrated semisolid vehicle is a cellulose polymer hydrogel.

According to a particularly effective preferred embodiment, said cellulose polymer is a hydroxypropylmethyl cellulose hydrogel. The hydroxypropylmethyl cellulose (HPMC), is a semi-synthetic derivative of cellulose without charges which forms a hydrogel of colloidal nature when it is dissolved in aqueous solution. The hydrogel viscosity increases with increasing concentration of HPMC in the medium and it can be influenced by the presence and amount of different additives.

In a particularly preferred embodiment, the hydroxypropylmethyl cellulose hydrogel further comprises glycerol. In the following described examples, it is shown that the glycerol existing in the hydroxypropylmethyl cellulose hydrogel allows to slow down the water loss under air exposure.

An embodiment of the invention is represented by the composition, the invention relates to, wherein said hydroxypropylmethyl cellulose hydrogel comprises hydroxypropylmethyl cellulose in a concentration ranging from 1% w/v to 5% w/v, preferably 3% w/v, and glycerol in a concentration ranging from 5% w/v to 20% w/v, preferably 10% w/v.

According to an embodiment, said hydroxypropylmethyl cellulose hydrogel comprises hydroxypropylmethyl cellulose in a concentration of 3% w/v, and glycerol in a concentration of 10% w/v.

In another embodiment, said silica sol-gel and said hydroxypropylmethyl cellulose hydrogel are therebetween in a ratio from 1000:1 to 1:10000, preferably from 50:1 to 1:1000 (w/w).

Additionally, in an embodiment according to any one of the herein described embodiments, said silica sol gel and said hydroxypropylmethyl cellulose hydrogel are therebetween in a ratio of 1:200 (w/w).

In an embodiment, the composition, the invention relates to, further comprises one or more excipients and/or carriers.

The excipients and the carriers usable for the formulation of the composition, and techniques for the formulation of said composition with said excipients and carriers, are known and easily accessible to the person skilled in the art.

In an embodiment, the composition, the invention relates to, is for topical use by controlled administration of said active ingredient.

In an additional embodiment, the composition according to any one of the herein described embodiments is for use as a medicament.

In a preferred embodiment, the composition, the invention relates to, is for use in the treatment of chronic ulcers, diabetic and diabetic foot ulcers.

Under chronic ulcer a continuous skin lesion is meant which does not heal and does not progress through the usual phases and usual healing time and the most common chronic ulcers include the lower limb ulcers, the diabetic ulcers and the pressure ulcers.

The authors of the present invention have surprisingly noted that the composition described in the present description resulted to be very useful even in improving the trophism of skin appendages, in particular associated to the skin lesions, chronic ulcers, diabetic ulcers and diabetic foot.

Therefore, in an embodiment, the composition, the invention relates to, is for use in the improvement of trophism of skin appendages in a method of treatment or prevention, for example, of skin lesions, such as for example chronic ulcers, diabetic ulcers and diabetic foot.

The present invention further relates to the not therapeutic use of the composition described in the present description according to any one of the herein described embodiments, for the improvement of the trophism of skin appendages.

Under improvement of trophism of skin appendages for example the favouring of hair regrowth is meant.

The present invention further relates to a method for the preparation of a composition comprising or consisting of a silica sol gel, wherein an active ingredient is incorporated in said silica sol gel, as defined in the claims.

Said silica sol gel obtained by the method, the invention relates to, is a wet silica sol gel.

The method, the invention relates to, comprises the following steps:

i) preparation of an activated sol;
ii) mixing said activated sol with a solution of active ingredient so as to obtain a wet silica sol-gel incorporating said active ingredient;
iii) washing the wet gel to replace the dispersing solution with a suitable buffer, after, or not, crushing it to generate a suspension of coarse wet gel particles;
iv) dilution of said sol-gel obtained at point iii) in a cellulose polymer hydrogel. In an embodiment, said step i) is carried out by hydrolyzing an alkoxysilane precursor with an acid treatment at a temperature from 0°C to 80°C, preferably from 4°C to 40°C, for at least 30 minutes.

According to an embodiment, said acid treatment is performed at a temperature comprised between 4°C and 40°C for 30 minutes.

The alkoxysilanes are organic compounds of general formula Si(OR)n, that is they are formed by four (equal or different) alkoxide groups bound to a silicon atom. The most common alkoxysilanes are tetraethoxysilane (TEOS) and tetramethoxysilane (TMOS). The alkylated derivatives of silanes, called alkyl alkoxysilanes, are very common. Even alkoxysilanes of polysilcates can be synthetized thus obtaining the polyalkoxysilanes.

The alkoxysilanes are used as precursors in the sol-gel process to produce high-quality glassy and ceramic materials.

According to an embodiment, said alkoxysilane precursor is selected among: tetramethoxysilane, tetraethoxysilane and/or diglyceroxylsilane.

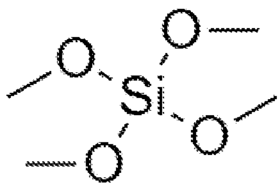

Tetramethoxysilane

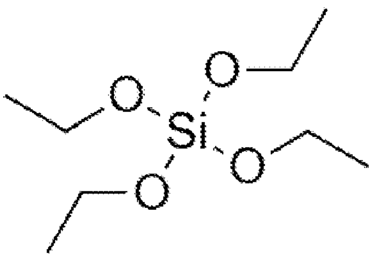

Tetraethoxysilane

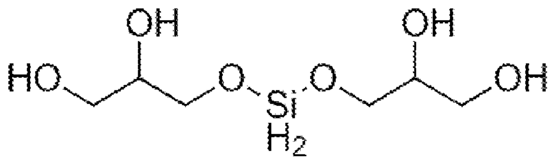

Diglyceroxylsilane

In a preferred embodiment, said precursor of alkoxysilane is tetramethoxysilane.

In an embodiment, the molar ratio between Si:HCl:$H_2O$ is 1:6x$10^{-6}$:1.25.

According to any one of the herein described embodiments, in an embodiment, said step ii) of the method, the invention relates to, is carried out by using a volume ratio between said solution of protein and said activated sol, ranging from 1.35:1 to 10.73:1, preferably circa 3.5:1

In an embodiment of the present invention, in the wet silica sol gel obtained according to step ii), the ratio between the silica ($SiO_2$) and the solution in which it is immersed ranges from 2% to 20% w/v, preferably from 4% to 15% w/v.

In an embodiment, said volume ratio between said solution of active ingredient and said activated sol is about 3.5:1.

In an embodiment of the present invention, said solution of active ingredient has a pH from 2 to 12, preferably 6.

In an additional embodiment the wet silica sol gel obtained according to step ii), is crushed manually or mechanically until obtaining a coarse suspension of the wet gel.

According to an additional embodiment, according to any one of the herein described embodiments, in the method, the invention relates to, said cellulose polymer hydrogel wherein the wet silica sol gel is diluted, is a hydroxypropylmethyl cellulose hydrogel comprising glycerol.

In a preferred embodiment, in said step iv) of the method, the invention relates to, said silica sol-gel obtained at point iii) and said hydroxypropylmethyl cellulose hydrogel are therebetween in a ratio from 1000:1 to 1:10.000, preferably from 50:1 to 1:1000.

In a following embodiment, said silica sol-gel obtained at point iii) of the method, and said hydroxypropylmethyl cellulose hydrogel are therebetween in a ratio 1:200.

In an embodiment of the method of the invention, said active ingredient is a biological macromolecule.

In an embodiment of the method described by the present invention, said active ingredient is a protein.

In a preferred embodiment, said protein is Serpin B3 or a fragment thereof or a variant thereof with the same function.

In any part of the present description and of the claims, the concentration designated with "w/v" represents the concentration "mass/volume", defined as grams of solute dissolved in 100 mL of solution.

In any part of the present description and of the claims, the concentration designated with "w/w" represents the concentration "mass/mass", defined as grams of solute dissolved in 100 grams of solution.

In conformity to Art. 170bis of the Italian law on patents it is declared that all experiments relating the cells were performed on cells available in commerce or obtained in our laboratories.

In any part of the present description and of the claims, the term comprising can be replaced by the term "consisting of".

Examples are reported hereinafter having the purpose of illustrating better the methods illustrated in the present description, such examples are in no way to be considered a limitation of the previous description and of the subsequent claims.

## Examples

### Materials and Methods

### Materials

2-mercaptoetanol (Merck, # 700-SC463253); 2-propanol (Sigma, # I9516); Ab Goat anti-Rabbit IgG, (H + L) HRP AP307P (EMD Millipore Corp., # 2842191); rabbit human anti-SB3 polyclonal Ab (Xeptagen S.p.A, Vega Park, Venezia); ascorbic acid ($C6H8O6$, Panreac, # 0000421452); boric acid ($H3BO3$, Sigma, # BCBP2177V); 37% hydrochloric acid (HCl, Scharlau, # AC07742500); 98% oxalic acid ($C2H2O4$, Sigma Aldrich, # S19352-016); concentrated sulphuric acid ($H2SO4$, Scharlau, # AC20671000); 40% acrylamide-bisacrylamide (Sigma, # 028K8220); bovine serum albumin (BSA, Sigma, # 078K0710); ammonium chloride ($NH4Cl$, Sigma Aldrich, # 254134); tetrahydrate molybdic ammonium ((NH4) MoO24 4H2O, Sigma Aldrich, # 125K0034); ammonium persulfate (APS, Sigma, # MKBX2380V); Bromophenol blue B8026-5G (Sigma Aldrich, # 097K3402); DMSO ($CH3SOCH3$, Carlo Erba, # P6M020077I); calcium chloride ($CaCl2$, Sigma Aldrich, # C1016); ECL Prime Western Blotting Detection Reagent (GE Healthcare, # 9995053); fluorescein isothiocyanate (FITC, Sigma, # 039K5310); glycerine; glycine (Plusone, # 17-1323-01); HEPES ($C8H18N2O4S$, Sigma Aldrich, # 018K54482); hydroxypropylmethyl cellulose (HPMC, Seppic, 90-SH-100000 SR); Kollidon 30 (BASF, # 63-0137); Electrophoresis marker C1992-1VL (Sigma, # 108K6401, MW: 8.000-222.000); methanol (MeOH, chemical products VWR, # 15G010506); sodium pentahydrate metasilicate ($Na2SiO3 \cdot 5H2O$, Sigma Aldrich, # 71746); 99% N, N, N', N'-tetramethylethylendiamine (TEMED, Sigma Aldrich, # STBH3450); Pronase by Streptomyces griseus (Roche Diagnostics, # 25551124); sodium chloride (NaCl, A.C.E.F., # H1339001); sodium dodecyl sulfate (SDS, Fluka, # 1315128-31107327); sodium phosphate monosodium dihydrate ($NaH2PO4$, A.C.E.F., # G2728003); tetramethyl orthosilicate (TMOS, Sigma Aldrich, # BCBN3603V); base Trizma (Sigma, # SLBM8522V); Tween 20 (A.C.E.F., # G6105003). The human SerpinB3 (SB3) was produced with techniques of genomic recombination in our laboratories, as previously described (Turato et al. Exp Biol Med 2011, 236:281-290), whereas the analogous mouse Serpinb3 was acquired by NZYTech (Lisbon, Portugal).

### Instrumentation

The spectroscopic analyses were performed with a UV Varian Cary 50 UV-Vis Spectrophotometer. Fluorescence was measured by using a JASCO FP-6200 spectrofluorometer. The circular dichroism spectra were recorded by using a JASCO J-810 spectropolarimeter. The FPLC analyses were performed by using the GE Healtcare AKT-A Purifier instrument with quality Superose 6 column prepared in a 10/300 mm Tricorn and Thermo-Scientific UV tec column. The studies about the release of proteins were performed by using Float-A-Lyzer G2 1-mL test tubes for dialysis by Spectrum Laboratories, Inc. For the gel wash a Hettich Universal 320-R centrifuge was used. For the sterilization an autoclave Timo was used. As thermostatic bath, SV 1422 model by Memmert GmbH was used. The electrophoresis on gel of polyacrylamide was performed by using a gel casting of Bio-Rad Mini-Protean System. The Western blot was performed on Millipore PVDF membranes (polyvinylidene fluoride). For acquiring the Western blot membranes, the Molecular Imager VersaDoc MP4000 was used. For the rheological analyses, the HAAKE Rotovisco viscosimeter was used. For analysing in real time the essays of proliferation, migration and cell invasion the xCELLigence DP instrument (ACEA, Biosciences, Inc., San Diego, CA, USA) was used. For evaluating the cell immunofluorescence, the Axiovert 200M microscope (Carl Zeiss Microscopy GmbH, Germania) was used.

**Preparation of sol-gel silica hydrogel**

All passages were performed in a sterile environment to prevent the microbial contamination. The gels (8% $SiO_2$ w/v) were obtained by tetramethoxysilane (TMOS) according to a procedure already described in Teoli D, Parisi L, Realdon N, Guglielmi M, Rosato A, Morpurgo M. Wet sol-gel derived silica for controlled release of proteins. J Control Release. 2006 Dec 1;116(3):295-303. doi: 10.1016/j.jconrel.2006.09.010. Epub 2006 Sep 26. PMID: 17097181.0,;Tonon G and Morpurgo M, "Sol-gel derived silica polymers for the sustained release of proteins" Patent WO2007115860A1; shortly, the partial hydrolysis of alkoxysilane precursor (activated sol) was at first promoted by an acid treatment at room temperature for 30 minutes by using HCl (molar ratios Si:HCl:$H_2O$ = 1: 6x10-6: 1,25). For each ml of wet silica sol-gel, 230.4 μl of activated sol, under delicate mild shaking, were mixed with 769.6 μl of protein solution in PBS+0.1% Kollidon 30. After gelling, the gel was crushed manually with a sterile spatula and the methanol released during the condensation phase was removed by extensive washing (3X) performed by adding 5 volumes of buffer (100 mM phosphate pH 6,0), centrifugation (1390 g, 1 min) and removal of supernatant. The product was used soon after preparation and kept at 4°C sealed with a minimum amount of buffer to avoid drying. For the in vivo administration it was diluted in a 3% sterile (metolose) HPMC hydrogel containing 10% glycerol.

**Structural conformation of SerpinB3**

The conformation of SerpinB3 (SB3) protein in solution and inside the wet silica sol gel was determined by means of circular dichroism. The analysis was performed at 25°C in a quartz cuvette with optical path of 1 mm and the following parameters were set: λ: 190-260 nm; bandwidth: 2 nm; response time: 16 s; sensitivity: standard; data pace: 0.1 nm; scanning speed: 10 nm/min; acquisitions: 2 (for white) and 4 (for proteins). The spectra in solution were recorded at 0.15 mg/mL SB3 in 100 mM phosphate pH 6.0. The spectra in wet gel (SB3 at 0.3 mg/mL gel) were recorded by the suspended suspension in an equal volume of buffer inside the quartz cuvette.

**Thermal stability of SerpinB3**

The melting temperature (t-melt) of protein in solution (0.15 mg/mL in 100 mM phosphate pH 6.0) and when trapped inside the matrix of wet silica gel sol-gel (0.3 mg/mL in gel, diluted 1:1 with 100 mM phosphate, pH 6.0) was determined by circular dichroism. CD spectra were acquired, repeated (between 200 and 260 nm with intervals of 0.1 nm) at intervals of 10°C in a temperature range between 40°C and 90°C, with a heating speed of 60°C/h. The thermal denaturation curves were obtained by showing the ellipticity average value for residue measured at 222 nm depending upon the analysis temperature.

**Stability to proteolysis of SerpinB3**

SB3 was analysed by Western blot (after 12% SDS PAGE) after its release from wet matrix of silica sol-gel previously incubated with the proteolytic pronase enzyme. In details, before the release assay, the wet matrix of silica sol-gel (200 μl) containing SB3 (300 μg/mL) was suspended in 500 μl of pronase (3 μg/mL- SB3/pronase w/w ratio = 40: 1) in HEPES 10 mM + NaCl 150 mM + $CaCl_2$ 10 mM pH 7.4. After 1 hour of incubation at 37°C, the gel was washed 6 times with phosphate 100 mM pH 6.0 to remove pronase and then incubated at 37°C in 10 mL of release buffer (buffer HEPES 100 mM + 0.1% BSA + 0.05% Tween 20pH 7.4). After 1 h, the release solution was analysed by SDS-PAGE and Western blot. The presence of SB3 on the membrane was detected by chemioluminescence (ECL) after incubation with rabbit anti-human SB3 antibody, and subsequently with goat anti-rabbit IgG marked with peroxidase (HRP). In the same way, the protein in solution was incubated with pronase with ratio SB3:pronase w/w = 40:1) for 1 h at 37°C and then analysed by Western blot.

**Release and functional analysis of SerpinB3**

200 μl of the prepared product containing SB3 in silica hydrogel were suspended in 10 mL of phosphate buffer 100 mM, 0.05% Tween 20, 0.1% BSA, pH 6.0 and incubated at 37°C under slight stirring. At defined time moments (0 h, 5 h and 24 h) the sample was centrifuged (1390 g) and a small portion of supernatant (250 μl) was removed by titration of SB3 and replaced with fresh buffer. The compound was then re-suspended in the supernatant by a slight vortex and the incubation at 37°C was continued until the subsequent moment. The concentration of SB3 in the supernatants was quantified by sandwich ELISA (kit HEPA Lisa, Xeptagen S.p.A, Vega Park, Venice) by following the manufacturer's instructions. Shortly, 100 μL of release solution 1:5 from gel with SB3 at concentration of 5 μg/mL or of release solution 1:50 from gel with SB3 at concentration 50 μg/mL were incubated for 1 hour at room temperature on plates coated with rabbit anti-human SB3 capture Ab (10 μg/mL in PBS, pH 7.4). Even the standard curve was included, obtained by dilution of SB3

recombinant from 16 to 0.25 ng/mL. All samples were tested in duplicate. After washing, the concentration of SB3 was detected by incubation with 100 μL of anti-SB3 secondary Ab conjugated with streptavidin HRP (0.5 μg/mL). The plate was developed with a solution of substrate 3,3', 5,5'-tetramethylbenzidin (TMB) ready for use. The reaction was interrupted with 1 mol/L of HCl (100 μL) and the absorbance at 450 nm was measured on a reader of microplates (Victor × 3; Perkin Elmer, Waltham, MA, USA).

The results were represented graphically by tracing the ratio between the amount of protein released in each time point (Mt) and the total protein (Mtot) incorporated in function of time. The released functional capability of SB3 was verified by scatter assay, as previously reported (Quarta et al. J Pathol 2010;221:343-356). In detail, canine kidney cells Madin-Darby (MDCK) ($5x10^3$ cells/well), were treated with two different dilutions of SB3 released by the wet silica microspheres for 5 and 24 hours. The cells were fixed in 4% formaldehyde, coloured with blue solution of Coomassie and the scattering effect was analysed by using the Axiovert 200M microscope (Carl Zeiss Microscopy GmbH, Germania). As positive control, SB3 recombinant at 200 ng/ml was used.

To support the rational of use of murine SB3 (mSB3) in the animal model, preliminary tests of the effect of this molecule on murine fibroblasts were performed. The fibroblasts of the dermal layer were insulated, as described previously (Fadini et al. 2010, Diabetes), by placing ~ 1 mm2 of skin plasters in a Petri plate with the dermis portion adhering to the plate bottom, in presence of medium DMEM 5 mM glucose (Sigma Aldrich) + 10 % FBS (Invitrogen, Carlsbad, CA, USA) and 1% L-glutamine/penicillin-streptomycin (Sigma Aldrich). The fibroblasts obtained from the explants were cultivated until reaching 80% of confluence and then subjected to plate passage. The cell proliferation in real time was performed by the xCELLigence DP instrument (ACEA, San Diego, USA) as described in the manufacturer's instruction manual. Shortly, 2,000 cells/well of mouse fibroblasts were sowed on E 16 plates and plated for 24 hours. They were then incubated with increasing amounts of mSB3 and the proliferation index was monitored up to 72 hours. Parallelly, an invasion test was performed by using the CIM plate of the xCELLigence system. As solid matrix Matrigel was used. 2,000 cells/well were sowed on CIM plate and treated with increasing amounts of mouse SB3. The cell index, which in this case represents the capability of cell invasion, was monitored every 15 minutes (figure 8).

**In vivo test**

The experiments on animals were approved by the Animal Care of University in Padova and the Italian Health Ministry. Diabetes was induced by a single intraperitoneal injection of 150 mg/kg of streptozotocin (STZ) (Sigma Aldrich) in citrate buffer 50 mmol/l, pH 4.5. Glycemia was measured with Glucocard G-meter (Menarini, Firenze, Italy); mice with glycemia ≥16.7 mmol/l (300 mg/dl) in at least two measurements within the first week were classified as diabetic and housed for 4 weeks with free access to food and water before performing the experiments. In order to create skin wounds, the mice were sedated with inhaled sodium isoflurane and the skin of the rear portion of the back was shaved with Veet cream and disinfected with 10% iodopovidone. Then, a wound having diameter of 4 mm was executed with a punch for biopsy (H-S Medical, Colton, CA, USA).

The wounds of animals were treated locally with 10 ng of SB3 formulated in 100 μL of phosphate buffer 100 mM + 0.1% Kollidon-30, pH 6.0, or 100 μL of metolose or 100 μL of metolose containing silica hydrogel (volume 1/200) with SB3 (200 μg/mL). As controls, metolose and silica hydrogel without SB3 diluted 200x in metolose were used. Each treatment was administered immediately after executing the wound. In the observation period no medication was applied to wounds. In order to evaluate the wound closing time and the hair restoration, pictures were taken daily and the wound area was quantified with ImageJ.

**Additional information**

**The methyl cellulose hydrogel does not influence the diffusion of proteins**

In a preliminary test it was evaluated if the use of hydrogel of 3% w/v HPMC as diluent for the suspension of silica nanoparticles influences the release of a protein incorporated therein. To this purpose, preparations constituted by silica hydrogel having incorporated the model protein BSA-FITC, suspended in an equal volume of release buffer or t 3% HPMC, were inserted in dialysis tubes (cut-off 100KDa) and immersed in PBST + 0.1% BSA at 37°C. At programmed time points the content of BSA-FITC was quantified in the acceptor solution by fluorescence spectroscopy. Figure 5 shows that the release profiles generated in presence or absence of HPLC hydrogel are almost superimposable, by demonstrating that the presence of HPMC has no impact on the release kinetics.

**The presence of glycerol in the methyl cellulose hydrogel slows down the water loss**

Several mixtures of 3% w/v methyl cellulose hydrogel (HPMC) and glycerol in ratios (w/w) ranging from 100: 5 and 100: 20 were prepared. The mixtures (about 1 g) were kept in open air, at room temperature, up to 24 h, and their weight

(w) was measured each hour for the first 5 h and then at 24 h. Their % water residual content of (RH2O%), excluding content of glycerol, was calculated by using the following formula: $RH2O\ \% = \left(\frac{w_x}{w_0}\right) * 100$ wherein w0 is the initial weight subtracted from the weight of the (not evaporating) glycerol fraction and wx is the weight at instant x, subtracted from the initial glycerol fraction.

**The methyl cellulose hydrogel (HPMC) alone and silica without SerpinB3 do not influence the wound healing**

In order to exclude the possibility of a matrix effect in the healing of wounds produced by the silica hydrogel vehicle/HPMC, the hydrogel of HPMC without SB3 and silica sol-gel was applied on wounds of diabetic and not diabetic mice, showing that the healing was not influenced by both treatments without SB3. It results evident that diabetes extends the healing time of wounds (figure 9).

## Claims

1. A composition for topical use comprising or consisting of a wet silica sol gel, wherein an active ingredient is incorporated in said wet silica sol gel, wherein said wet silica sol gel is diluted in a cellulose polymer hydrogel.

2. The composition according to claim 1, **characterized in that** it has an amount of water in said gel from 76% to 98% w/v, preferably from 80% to 94% w/v.

3. The composition according to claims 1 or 2, wherein said active ingredient is a protein and/or another type of biological macromolecule and/or a peptide, preferably said active ingredient is physically incorporated in said wet silica sol gel.

4. The composition according to claims 1 to 3, wherein said active ingredient is Serpin B3 protein or a fragment thereof or a variant thereof with the same function.

5. The composition according to claims 1 to 4, wherein the ratio between $SiO_2$ and the solution in which it is immersed is in a range from 2 to 20%, preferably from 4% to 15% w/v, and/or wherein said active ingredient is present in a concentration in a range from 0.005 to 10.0 mg/ml, in particular said concentration relates to the final formulation.

6. The composition according to anyone of claims 1 to 5, wherein said cellulose polymer is hydroxypropylmethyl cellulose (HPMC), preferably wherein said hydroxypropylmethyl cellulose hydrogel further comprises glycerol.

7. The composition according to claim 6, wherein said hydroxypropylmethyl cellulose hydrogel comprises hydroxypropylmethyl cellulose in a concentration ranging from 1% w/v to 5% w/v, preferably 3% w/v, and glycerol in a concentration ranging from 5% w/v to 20% w/v, preferably 10% w/v, preferably wherein said hydroxypropylmethyl cellulose hydrogel comprises hydroxypropylmethyl cellulose in a concentration of 3% w/v, and glycerol in a concentration of 10% w/v, and/or wherein said silica gel and said hydroxypropylmethyl cellulose hydrogel are therebetween in a ratio from 1000:1 to 1:10000, preferably from 50:1 to 1:1000 v/v, preferably in a ratio of 1:200 v/v.

8. The composition according to claims 1 to 7 for use as a medicament.

9. The composition according to claims 4 to 8 for use in the prevention or treatment of chronic ulcers, preferably diabetic and diabetic foot ulcers, or for the improvement of the trophism of skin appendages in a method of treatment and prevention of a pathological state, preferably of chronic ulcers, diabetic ulcers and diabetic foot.

10. A method for the preparation of a composition comprising or consisting of a wet silica sol gel wherein an active ingredient is incorporated in said wet silica sol gel, comprising the following steps:

    i) preparation of an activated sol;
    ii) mixing said activated sol with a solution of an active ingredient so as to obtain a wet silica sol-gel incorporating said active ingredient;
    iii) washing the wet gel to replace the dispersing solution with a suitable buffer, after, or not, crushing it to generate a suspension of coarse wet gel particles;
    iv) dilution of said sol-gel obtained at point iii) in a cellulose polymer hydrogel.

11. The method according to claim 10, wherein said step i) is carried out by hydrolyzing an alkoxysilane precursor with an acid treatment at a temperature from 0°C to 80°C, preferably from 4°C to 40°C, for at least 30 minutes, preferably wherein said alkoxysilane precursor is selected among: tetramethoxysilane, tetraethoxysilane and/or diglyceroxylsilane.

12. The method according to claim 10 or 11, wherein said step ii) is carried out by using a volume ratio between said solution of active ingredient and said activated sol ranging from 1.35:1 to 10.73:1, preferably 3.5:1, preferably wherein said solution of active ingredient has a pH from 2 to 12, preferably 6.

13. The method according to claims 10 to 12, wherein said cellulose polymer hydrogel is a hydroxypropylmethyl cellulose hydrogel comprising glycerol.

14. The method according to claims 10 to 13, wherein in said step iv), said silica sol gel obtained at point iii) and said hydroxypropylmethyl cellulose hydrogel are therebetween in a ratio from 1000:1 a 1:10000, preferably from 50:1 to 1:1000 (v/v).

15. The method according to claims 10 to 14, wherein said active ingredient is a protein and/or another type of biological macromolecule and/or a peptide, preferably wherein said active ingredient is Serpin B3 protein or a fragment thereof or a variant thereof with the same function.

## Patentansprüche

1. Zusammensetzung zur äußeren Anwendung, umfassend oder bestehend aus einem feuchten Silica-Sol-Gel, wobei ein Wirkstoff in das feuchte Silica-Sol-Gel eingearbeitet ist, wobei das feuchte Silica-Sol-Gel in einem CellulosePolymer-Hydrogel verdünnt ist.

2. Zusammensetzung nach Anspruch 1, **dadurch gekennzeichnet, dass** sie eine Wassermenge in dem Gel von 76 bis 98 Gew.-/Vol.-%, vorzugsweise von 80 bis 94 Gew.-/Vol.-% aufweist.

3. Zusammensetzung nach Anspruch 1 oder 2, wobei der Wirkstoff ein Protein und/oder eine andere Art von biologischem Makromolekül und/oder ein Peptid ist, vorzugsweise der Wirkstoff in das feuchte Silica-Sol-Gel physikalisch eingearbeitet ist.

4. Zusammensetzung nach den Ansprüchen 1 bis 3, wobei der Wirkstoff Serpin B3-Protein oder ein Fragment davon oder eine Variante davon mit derselben Funktion ist.

5. Zusammensetzung nach den Ansprüchen 1 bis 4, wobei das Verhältnis zwischen $SiO_2$ und der Lösung, in die es eingetaucht ist, in einem Bereich von 2 bis 20, vorzugsweise von 4 bis 15 Gew.-/Vol.-%, liegt, und/oder wobei der Wirkstoff in einer Konzentration in einem Bereich von 0,005 bis 10,0 mg/ml vorliegt, insbesondere die Konzentration sich auf die endgültige Formulierung bezieht.

6. Zusammensetzung nach einem der Ansprüche 1 bis 5, wobei das Cellulosepolymer Hydroxypropylmethylcellulose (HPMC) ist, vorzugsweise wobei das Hydroxypropylmethylcellulose-Hydrogel ferner Glycerin umfasst.

7. Zusammensetzung nach Anspruch 6, wobei das Hydroxypropylmethylcellulose-Hydrogel Hydroxypropylmethylcellulose in einer Konzentration in einem Bereich von 1 Gew.-/Vol.-% bis 5 Gew.-/Vol.-%, vorzugsweise 3 Gew.-/Vol.-%, und Glycerin in einer Konzentration von 5 Gew.- /Vol.-% bis 20 Gew.-/Vol.-%., vorzugsweise 10 Gew.-/Vol.-%, umfasst, vorzugsweise wobei das Hydroxypropylmethylcellulose-Hydrogel Hydroxypropylmethylcellulose in einer Konzentration von 3 Gew.-/Vol.-% und Glycerin in einer Konzentration von 10 Gew.-/Vol.-% umfasst, und/oder wobei das Silicagel und das Hydroxypropylmethylcellulose-Hydrogel untereinander in einem Verhältnis von 1000 : 1 bis 1 : 10000, vorzugsweise von 50 : 1 bis 1 : 1000 Vol./Vol., vorzugsweise in Anteilen von 1 : 200 Vol./Vol. vorliegen.

8. Zusammensetzung nach den Ansprüchen 1 bis 7 zur Verwendung als ein Medikament.

9. Zusammensetzung nach den Ansprüchen 4 bis 8 zur Verwendung bei der Vorbeugung oder Behandlung von chronischen Geschwüren, vorzugsweise diabetischen und diabetischen Fußgeschwüren, oder für die Verbesserung des Trophismus von Hautanhangsgebilden in einem Verfahren für die Behandlung und Vorbeugung eines pathologischen Zustands, vorzugsweise von chronischen Geschwüren, diabetischen Geschwüren und diabetischem Fuß.

**10.** Verfahren für die Herstellung einer Zusammensetzung, umfassend oder bestehend aus einem feuchten Silica-Sol-Gel, wobei ein Wirkstoff in das feuchte Silica-Sol-Gel eingearbeitet ist, umfassend die folgenden Schritte:

i) Herstellung eines aktivierten Sols;
ii) Mischen des aktivierten Sols mit einer Lösung eines Wirkstoffs, um ein feuchtes Silica-Sol-Gel zu erhalten, das den Wirkstoff einarbeitet;
iii) Waschen des feuchten Gels, um die Dispersionslösung durch einen geeigneten Puffer zu ersetzen, nach oder ohne Zerkleinern desselben, um eine Suspension aus groben Partikeln des nassen Gels zu erzeugen;
iv) Verdünnung des in Punkt iii) erhaltenen Sol-Gels in einem Cellulosepolymer-Hydrogel.

**11.** Verfahren nach Anspruch 10, wobei der Schritt i) durch Hydrolysieren eines Alkoxysilan-Vorläufers mit einer Säure-Behandlung bei einer Temperatur von 0 °C bis 80 °C, vorzugsweise von 4 °C bis 40 °C, für mindestens 30 Minuten durchgeführt wird, vorzugsweise wobei der Alkoxysilan-Vorläufer ausgewählt ist von: Tetramethoxysilan, Tetraethoxysilan und/oder Diglyceroxylsilan.

**12.** Verfahren nach Anspruch 10 oder 11, wobei der Schritt ii) durch Verwenden eines Volumenverhältnisses zwischen der Lösung des Wirkstoffs und dem aktivierten Sol in einem Bereich von 1,35 : 1 bis 10,73 : 1, vorzugsweise 3,5 : 1, durchgeführt wird, vorzugsweise wobei die Lösung des Wirkstoffs einen pH-Wert von 2 bis 12, vorzugsweise 6, aufweist.

**13.** Verfahren nach den Ansprüchen 10 bis 12, wobei das Cellulosepolymer-Hydrogel ein Hydroxypropylmethylcellulose-Hydrogel ist, umfassend Glycerin.

**14.** Verfahren nach den Ansprüchen 10 bis 13, wobei in dem Schritt iv) das in Punkt iii) erhaltene Silica-Sol-Gel und das Hydroxypropylmethylcellulose-Hydrogel untereinander in einem Verhältnis von 1000 : 1 bis 1 : 10000, vorzugsweise von 50 : 1 bis 1 : 1000 Vol./Vol. stehen.

**15.** Verfahren nach den Ansprüchen 10 bis 14, wobei der Wirkstoff ein Protein und/oder eine andere Art von biologischem Makromolekül und/oder ein Peptid ist, vorzugsweise wobei der Wirkstoff Serpin B3-Protein oder ein Fragment davon oder eine Variante davon mit derselben Funktion ist.

## Revendications

**1.** Composition pour utilisation topique comprenant ou constituée d'un sol-gel de silice humide, dans laquelle un ingrédient actif est incorporé dans ledit sol-gel de silice humide, dans laquelle ledit sol-gel de silice humide est dilué dans un hydrogel de polymère de cellulose.

**2.** Composition selon la revendication 1, **caractérisée en ce qu'**elle a une quantité d'eau dans ledit gel allant de 76 % à 98 % en poids/volume, de préférence de 80 % à 94 % en poids/volume.

**3.** Composition selon les revendications 1 ou 2, dans laquelle ledit ingrédient actif est une protéine et/ou un autre type de macromolécule biologique et/ou un peptide, de préférence ledit ingrédient actif est physiquement incorporé dans ledit sol-gel de silice humide.

**4.** Composition selon les revendications 1 à 3, dans laquelle ledit ingrédient actif est une protéine Serpine B3 ou un fragment de celle-ci ou un variant de celle-ci avec la même fonction.

**5.** Composition selon les revendications 1 à 4, dans laquelle le rapport entre $SiO_2$ et la solution dans laquelle il est immergé est dans une plage allant de 2 à 20 %, de préférence de 4 % à 15 % en poids/volume, et/ou dans laquelle ledit ingrédient actif est présent en une concentration dans une plage allant de 0,005 à 10,0 mg/ml, en particulier ladite concentration se rapporte à la formulation finale.

**6.** Composition selon l'une quelconque des revendications 1 à 5, dans laquelle ledit polymère de cellulose est de l'hydroxypropylméthylcellulose (HPMC), de préférence dans laquelle ledit hydrogel d'hydroxypropylméthylcellulose comprend en outre du glycérol.

7. Composition selon la revendication 6, dans laquelle ledit hydrogel d'hydroxypropylméthylcellulose comprend de l'hydroxypropylméthylcellulose en une concentration allant de 1 % en poids/volume à 5 % en poids/volume, de préférence 3 % en poids/volume, et du glycérol en une concentration allant de 5 % en poids/volume à 20 % en poids/volume, de préférence 10 % en poids/volume, de préférence dans laquelle ledit hydrogel d'hydroxypropylméthylcellulose comprend de l'hydroxypropylméthylcellulose en une concentration de 3 % en poids/volume, et du glycérol en une concentration de 10 % en poids/volume, et/ou dans laquelle ledit gel de silice et ledit hydrogel d'hydroxypropylméthylcellulose sont entre eux dans un rapport allant de 1000:1 à 1:10000, de préférence de 50:1 à 1:1000 volume/volume, de préférence dans un rapport de 1:200 volume/volume.

8. Composition selon les revendications 1 à 7 pour utilisation en tant que médicament.

9. Composition selon les revendications 4 à 8 pour utilisation dans la prévention ou le traitement d'ulcères chroniques, de préférence diabétiques et d'ulcères du pied diabétique, ou pour l'amélioration du trophisme d'annexes cutanées dans un procédé de traitement et de prévention d'un état pathologique, de préférence d'ulcères chroniques, d'ulcères diabétiques et de pied diabétique.

10. Procédé destiné à la préparation d'une composition comprenant ou constituée d'un sol-gel de silice humide dans lequel un ingrédient actif est incorporé dans ledit sol-gel de silice humide, comprenant les étapes suivantes :

i) préparation d'un sol activé ;
ii) mélange dudit sol activé avec une solution d'un ingrédient actif de façon à obtenir un sol-gel de silice humide incorporant ledit ingrédient actif ;
iii) lavage du gel humide pour remplacer la solution de dispersion par un tampon approprié, après, ou non, son concassage pour générer une suspension de particules de gel humide à gros grains ;
iv) dilution dudit sol-gel obtenu au point iii) dans un hydrogel de polymère de cellulose.

11. Procédé selon la revendication 10, dans lequel ladite étape i) est effectuée par hydrolyse d'un précurseur d'alcoxysilane avec un traitement acide à une température allant de 0 °C à 80 °C, de préférence de 4 °C à 40 °C, pendant au moins 30 minutes, de préférence dans lequel ledit précurseur d'alcoxysilane est choisi parmi : tétraméthoxysilane, tétraéthoxysilane et/ou diglycéroxylsilane.

12. Procédé selon la revendication 10 ou 11, dans lequel ladite étape ii) est effectuée en utilisant un rapport volumique entre ladite solution d'ingrédient actif et ledit sol activé allant de 1,35:1 à 10,73:1, de préférence 3,5:1, de préférence dans lequel ladite solution d'ingrédient actif a un pH allant de 2 à 12, de préférence de 6.

13. Procédé selon les revendications 10 à 12, dans lequel ledit hydrogel de polymère de cellulose est un hydrogel d'hydroxypropylméthylcellulose comprenant du glycérol.

14. Procédé selon les revendications 10 à 13, dans lequel dans ladite étape iv), ledit sol-gel de silice obtenu au point iii) et ledit hydrogel d'hydroxypropylméthylcellulose sont entre eux dans un rapport allant de 1000:1 à 1:10000, de préférence de 50:1 à 1:1000 (volume/volume).

15. Procédé selon les revendications 10 à 14, dans lequel ledit ingrédient actif est une protéine et/ou un autre type de macromolécule biologique et/ou un peptide, de préférence dans lequel ledit ingrédient actif est une protéine Serpine B3 ou un fragment de celle-ci ou un variant de celle-ci avec la même fonction.

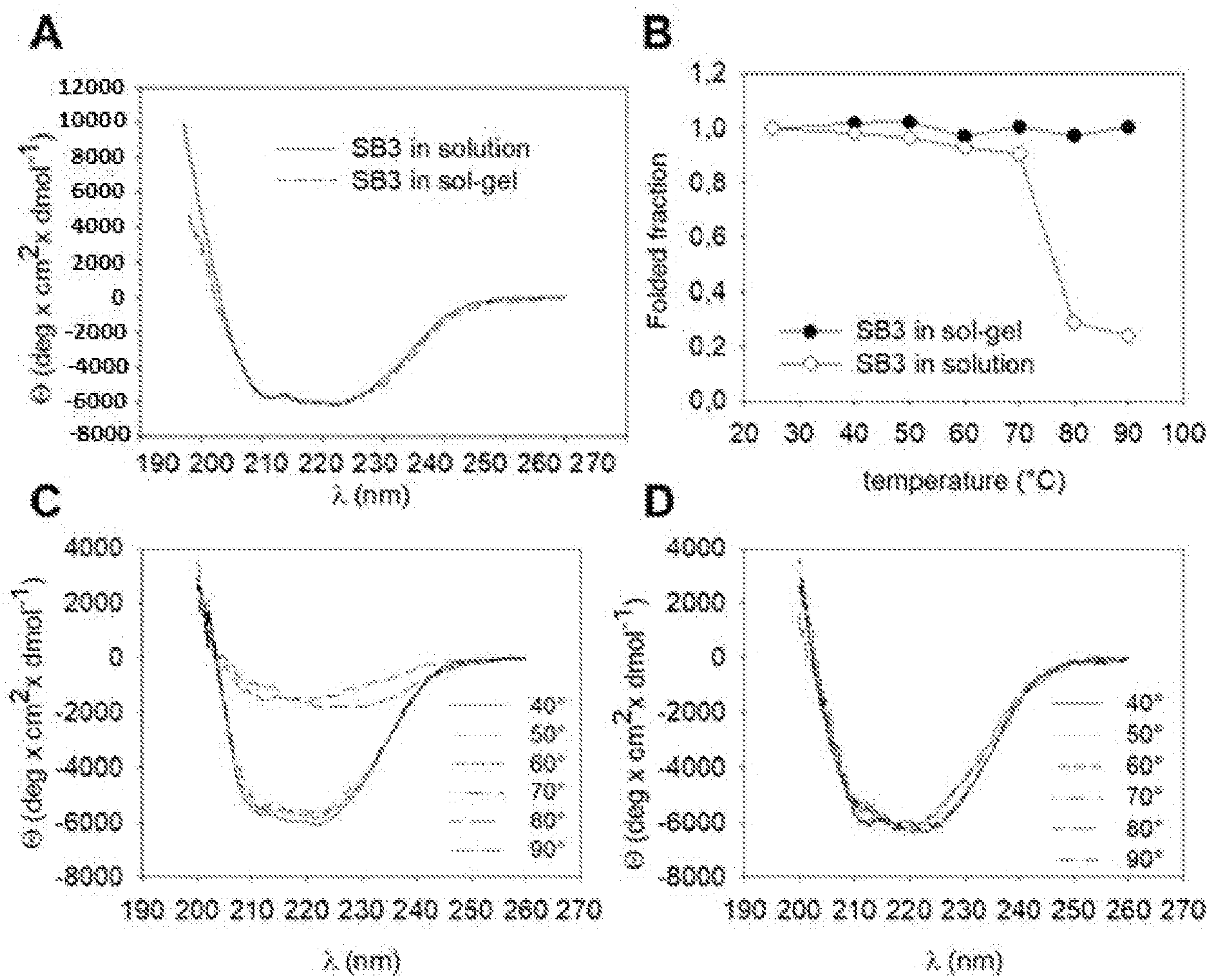


Figure 1

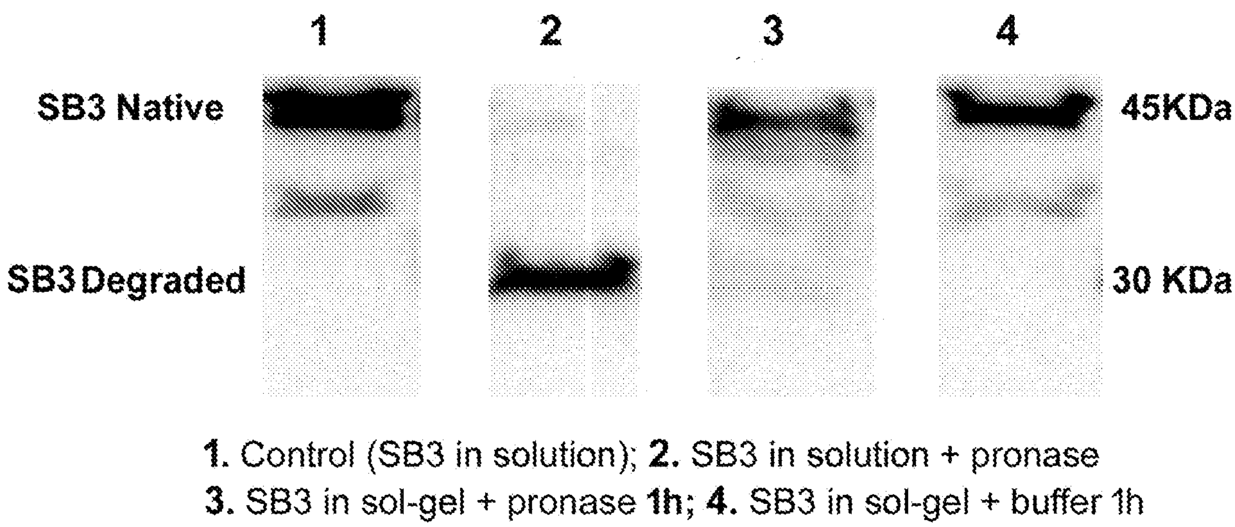


Figure 2

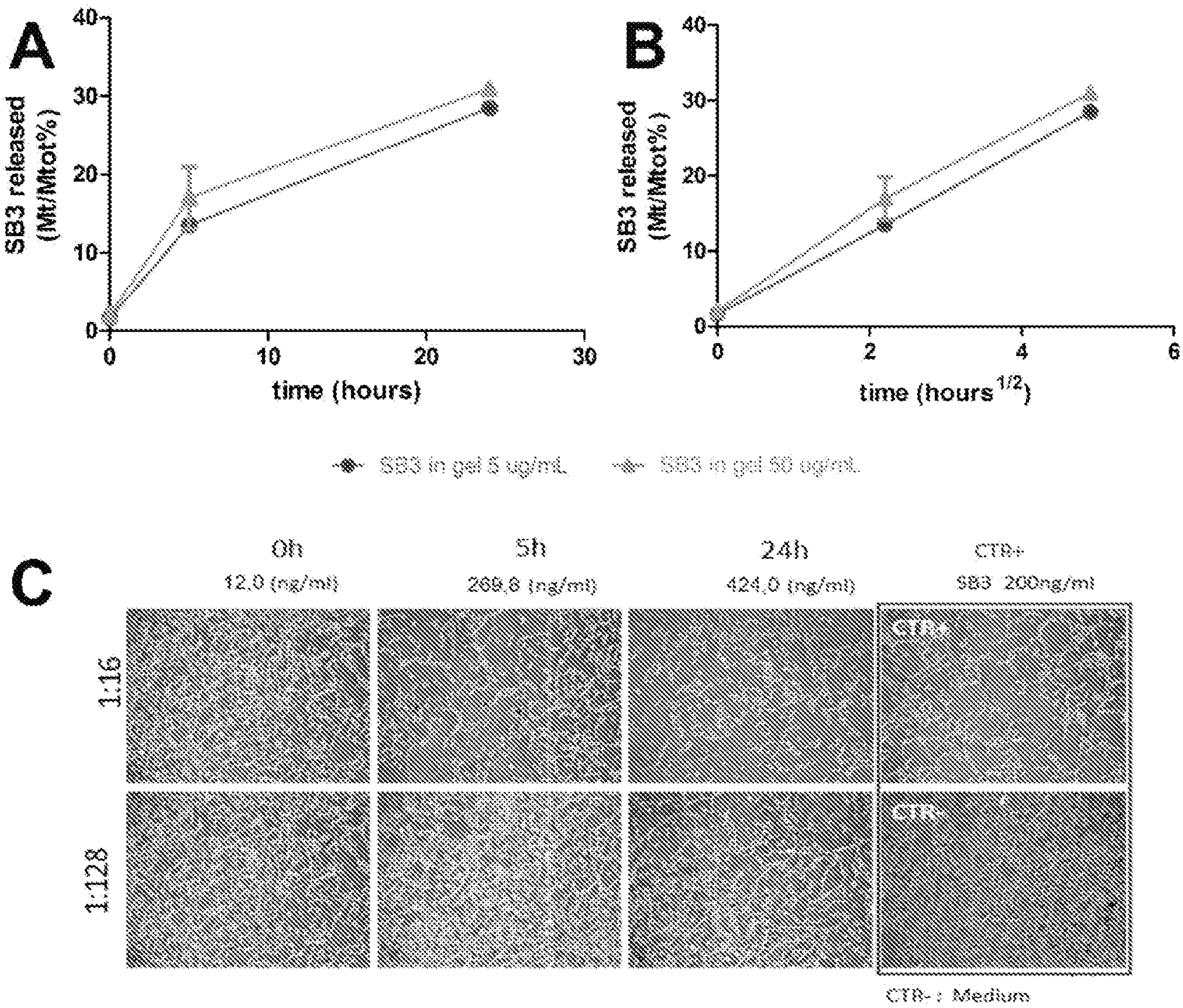
A
B
SB3 released (Mt/Mtot%)
time (hours)
time (hours$^{1/2}$)
SB3 in gel 5 ug/mL
SB3 in gel 50 ug/mL
C
0h
12,0 (ng/ml)
5h
269,8 (ng/ml)
24h
424,0 (ng/ml)
CTR+
SB3 200ng/ml
1:16
1:128
CTR+
CTR-
CTR- : Medium

Figure 3

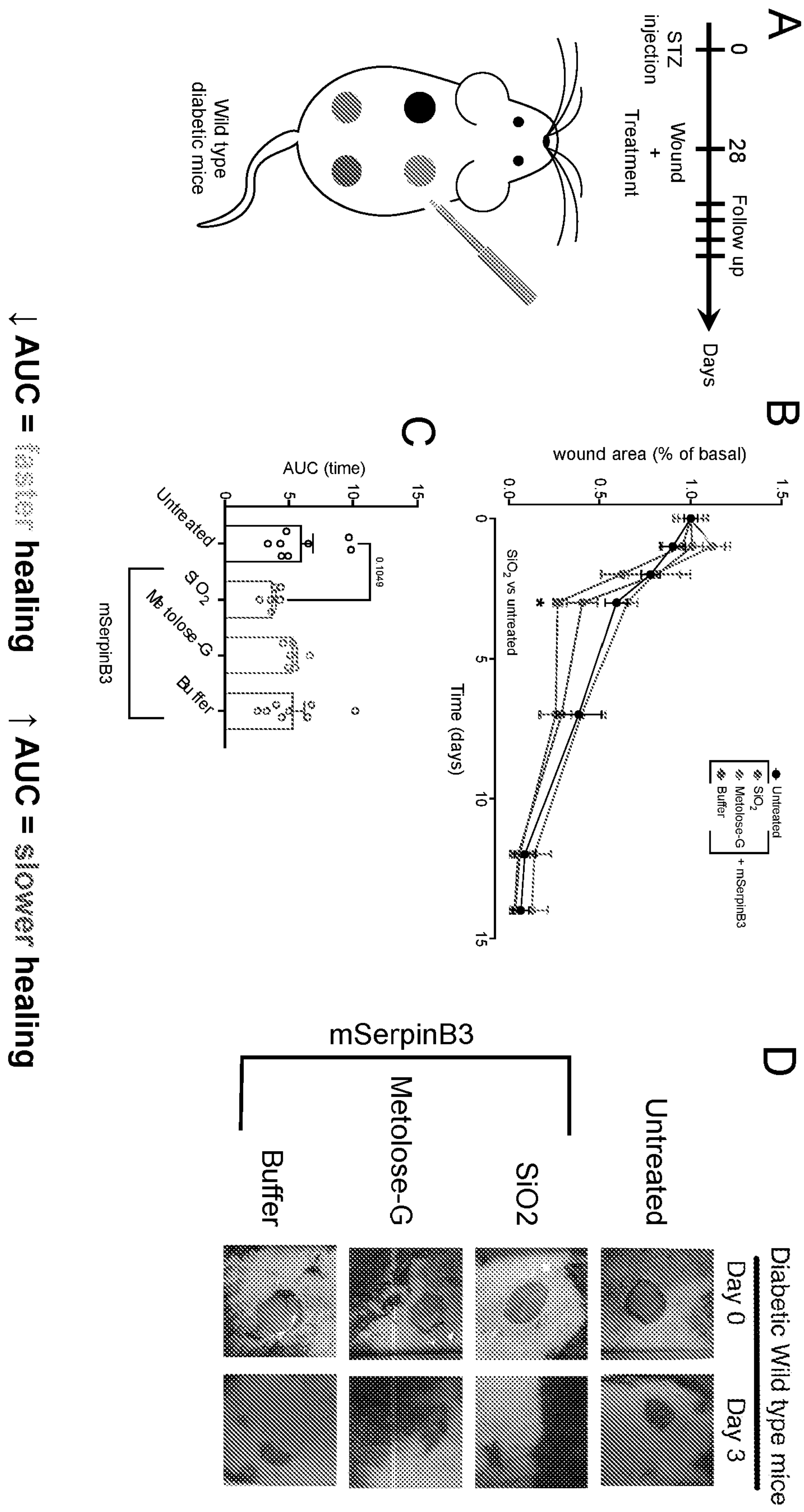
A
0
STZ injection
28
Wound + Treatment
Follow up
Days
Wild type diabetic mice
B
wound area (% of basal)
Time (days)
SiO2 vs untreated
Untreated
SiO2
Metolose-G
Buffer
+ mSerpinB3
C
AUC (time)
0.1049
Untreated
SiO2
Metolose-G
Buffer
mSerpinB3
↓ AUC = faster healing
↑ AUC = slower healing
D
Diabetic Wild type mice
Day 0
Day 3
Untreated
SiO2
Metolose-G
Buffer
mSerpinB3

Figure 4

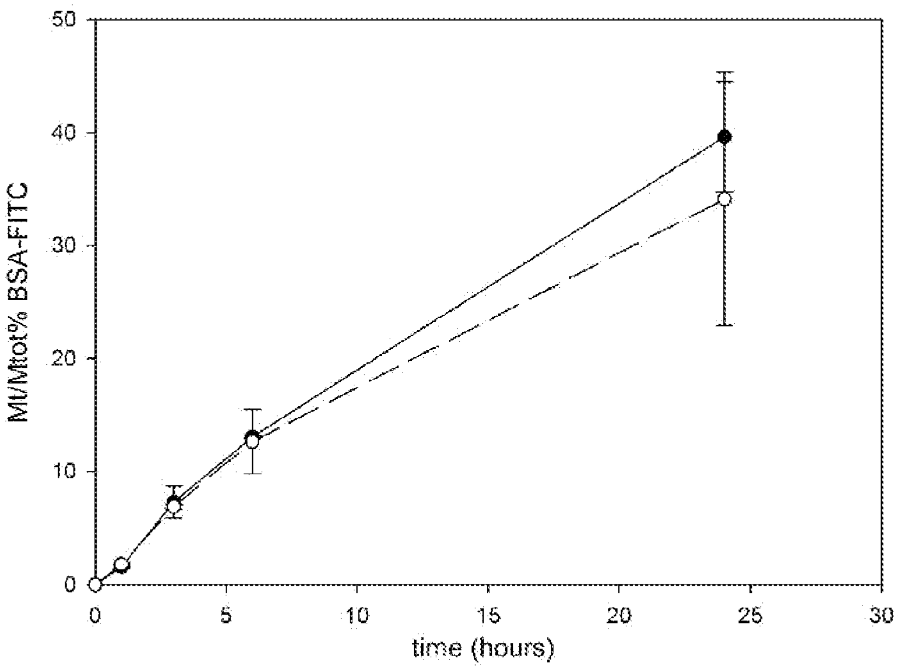


Figure 5

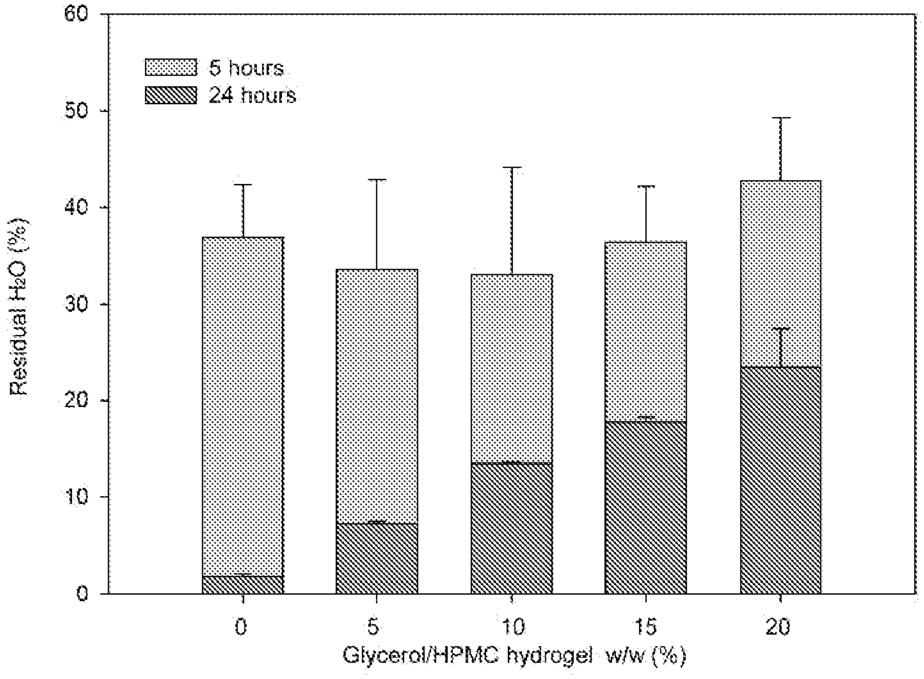


Figure 6

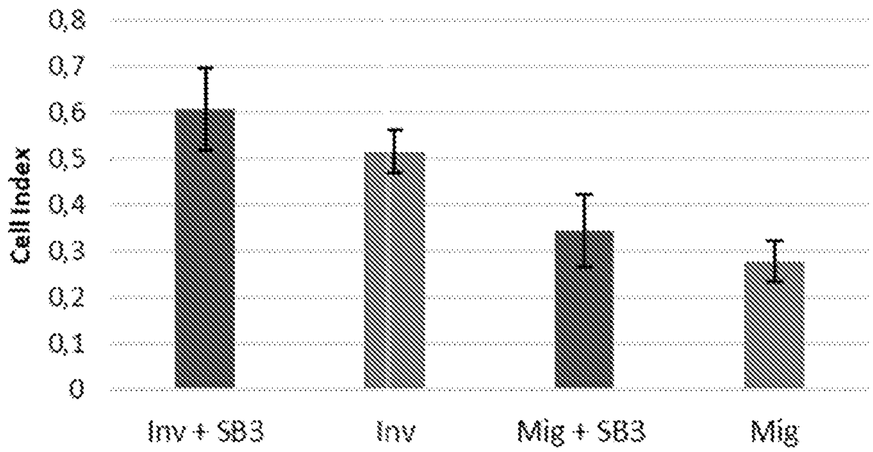


Figure 7

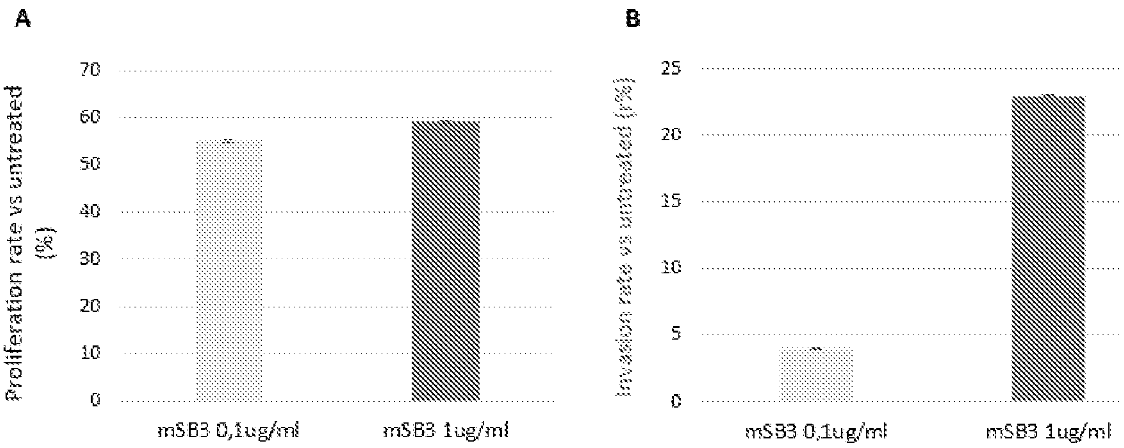


Figure 8

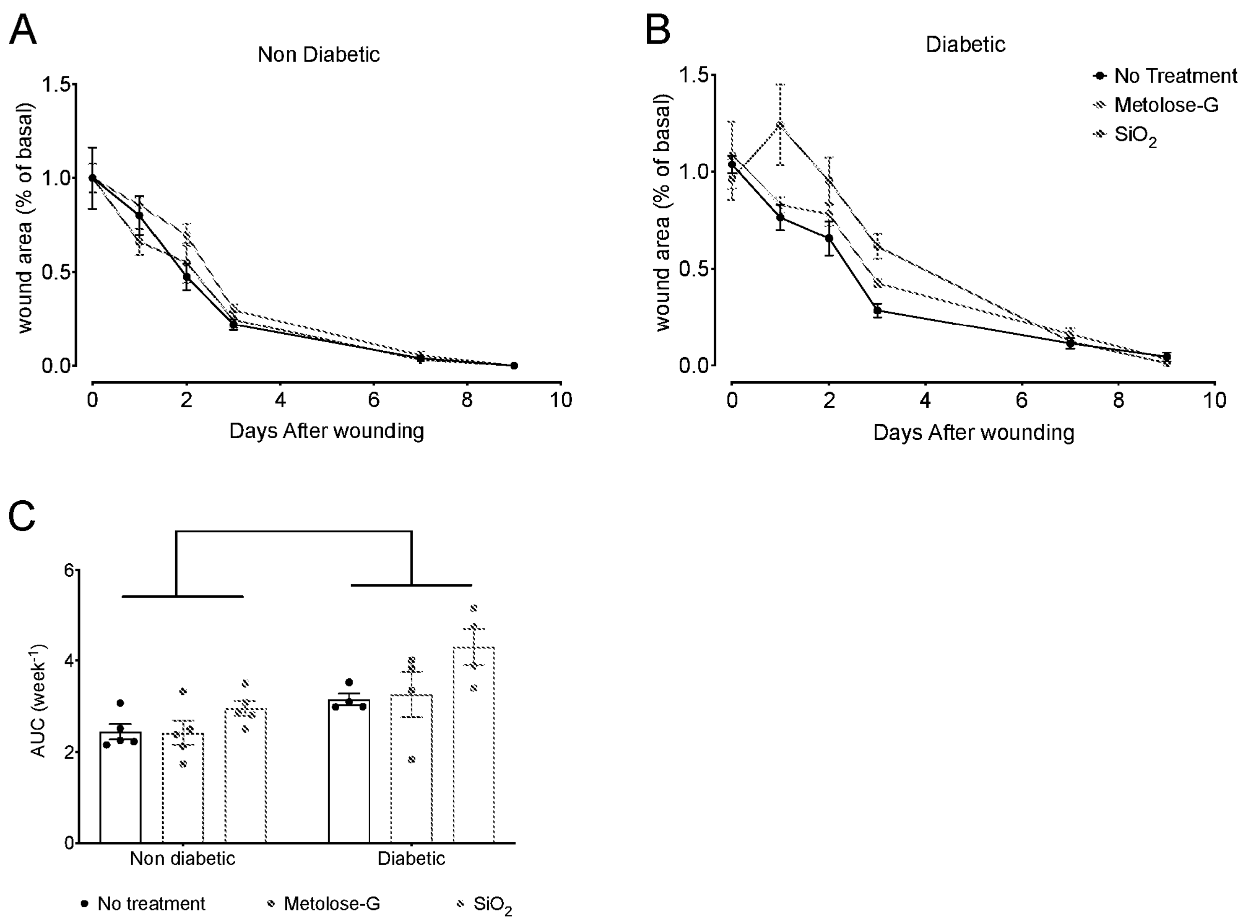
A
Non Diabetic
wound area (% of basal)
1.5
1.0
0.5
0.0
0
2
4
6
8
10
Days After wounding
B
Diabetic
No Treatment
Metolose-G
SiO2
wound area (% of basal)
1.5
1.0
0.5
0.0
0
2
4
6
8
10
Days After wounding
C
AUC (week-1)
6
4
2
0
Non diabetic
Diabetic
No treatment
Metolose-G
SiO2

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007115860 A1


### Non-patent literature cited in the description


- **AUFENVENNE K et al.** *Am J Hum Genet.*, 2013, vol. 93 (4), 620-30
- **FAISAL RAZA**. *Pharmaceutics*, 2018, vol. 10 (1), 16
- **ALLAN S.HOFFMAN**. *Advanced Drug Delivery Reviews*, 2012, vol. 64, 18-23
- **SUSANA SIMÕES et al.** *Journal of Biomaterials and Nanobiotechnology*, 2012, vol. 3, 185-199
- **KATO et al.** *Anticancer Res.*, 1996, vol. 16 (4B), 2149-2153
- **TURATO et al.** *Exp Biol Med*, 2011, vol. 236, 281-290
- **TEOLI D** ; **PARISI L** ; **REALDON N** ; **GUGLIELMI M** ; **ROSATO A** ; **MORPURGO M**. Wet sol-gel derived silica for controlled release of proteins. *J Control Release*, 26 September 2006, vol. 116 (3), 295-303
- **QUARTA et al.** *J Pathol*, 2010, vol. 221, 343-356
- **FADINI et al.** *Diabetes*, 2010